# EUROPEAN PATENT APPLICATION

(11) **EP 4 092 468 A1**
(43) Date of publication of application: **23.11.2022**
(21) Application number: 21174167.3
(22) Date of filing: 17.05.2021
(51) Int. Cl.: G02B 21/30

(54) **CONTROLLING SYSTEM AND EXAMINATION SYSTEM FOR MICROSCOPIC EXAMINATION OF A SAMPLE AND CORRESPONDING METHODS**

(71) Applicant: Leica Microsystems CMS GmbH, 35578 Wetzlar (DE)
(72) Inventor: CHRIST, Stefan, 35641 Schöffengrund (DE)
(74) Representative: DehnsGermany Partnerschaft von Patentanwälten

(57) **Abstract**

The present invention relates to a controlling system for operating an examination system (130) for microscopic examination of a sample (120), comprising a microscope (100), an incubation environment conditioning unit (110) connected to said microscope (100), and a user interface (142), said microscope (100) comprising: illumination optics (118), a microscope stage (116), imaging optics (124), a sample chamber (106) to receive the sample (120), a microscope interface for connection of the incubation environment conditioning unit (110) to the sample chamber (106), and an imaging optics chamber (122) separated from the sample chamber (106) and enclosing the imaging optics (124), the examination system (130) providing an incubation mode in which the sample chamber (106) is incubated by supply of an incubation atmosphere generated by said incubation environment conditioning unit (110), wherein the controlling system (140) is configured to: receiving a target setpoint (Tₛ**, CO₂**) of at least one examination parameter (Ts, CO₂), upon user input via said user interface (142); selecting, based on the received at least one target setpoint (Tₛ**, CO₂**), predefined adjustment setpoints for at least one incubation environment parameter (RH, CO₂) of the incubation mode and for at least one microscope parameter; and operating the incubation environment conditioning unit (110) and the microscope (100) based on the selected adjustment setpoints, and to such examination system (130).

## Description

### Technical Field

The present invention essentially relates to a controlling system for operating an examination system configured for microscopic examination of a sample, to such an examination system, to a method for microscopic examination, and to a method for operating an examination system.

### Background

Especially in the field of microscopic examination of living samples like cells, it is of great interest to keep the sample as long as possible under favourable and stress-free environmental conditions. To this end, incubators can be used for generating a microclimate adapted to the sample to be examined. Incubators can be distinguished in stage top incubators, on the one hand, and cage incubators, on the other hand.

Cage incubators are, typically, mounted to a standard microscope, which can be an upright microscope or an inverted microscope, and comprise a large climatic chamber covering the main parts of the microscope, such as the objective revolver, the microscope stage including the sample carrier and a condenser, such that a large volume needs to be incubated. Access to the working area for placing or manipulating the sample is provided inside the cage incubator and can be reached through dedicated openings in the cage incubator walls. Especially due to these handling openings, the dimensions of the cage incubator are so large that it protrudes significantly beyond the microscope stand. Therefore, it is not possible to equip a microscope with a cage incubator in a space-saving manner. On the other hand, a stage top incubator provides a small volume to be incubated as the stage top incubator only encloses the sample itself and is placed onto the microscope stage. Even if a stage top incubator has minimum space requirements, access to the sample is minimal since the sample is surrounded by a sealed box, which has to be opened, thus, destroying the incubation atmosphere within the box. Access to the sample to introduce additional equipment for manipulation of the sample is difficult if not impossible due to the tight space restrictions of a stage top incubator module. While a cage incubator has a high energy and gas consumption, a stage top incubator provides a small closed incubated room including connected supply conduits for supplying the desired incubation atmosphere. On the other hand, the stage top incubator module is able to quickly equilibrate any disturbance of the incubation atmosphere (e.g. after opening the module) or reach the desired setup as the exchanged air volume per time of a cage incubator usually exceeds that of a stage top incubator.

A stage top incubator allows close control of the direct incubation environment surrounding the sample, the regulated volume being reduced to a minimum, which allows quick changes to the incubation environment However, access to the sample itself is very limited and, hitherto, the stage top solution adds significant complexity and a high price to the customer system. On the other hand, the cage incubator allows easier access to the sample, but it is slow when it comes to changing environmental conditions or reaching a set point of a predefined incubation atmosphere.

When examining a sample using such microscope and incubator, different temperatures and other parameters have to be set by the user to specific values in order to achieve a desired sample temperature.

### Summary

In view of the situation described above, there is a need for an improved incubation solution in microscopy. According to embodiments of the invention, a controlling system, an examination system, a method for microscopic examination and a method for operating an examination system with the features of the independent claims are proposed. Advantageous further developments form the subject matter of the dependent claims and of the subsequent description.

An embodiment of the invention relates to an examination system configured for microscopic examination of a sample, in particular a living sample like a cell. Note that examination of a sample also can comprise imaging of such sample. The examination system comprises a microscope, an incubation environment conditioning unit connected to said microscope, and a user interface. Such user interface preferably comprises at least one of a display and input devices. For example, the user interface can comprise or be a touch display. Further, such examination system comprises a controlling system for operating the examination system, which is also a subject of an embodiment of the present invention. In the following, the controlling system and the examination system as well as the corresponding operating method will be described together.

Said microscope comprises illumination optics with, e.g., a light source and a condenser, a microscope stage (on which the sample is to be placed), and an imaging optics with, e.g., an objective and an imaging detector. Said microscope further comprises a sample chamber configured to receive the sample (preferably, the sample side of the microscope stage is part of such sample chamber), a microscope interface configured for connection of the incubation environment conditioning unit to the sample chamber, and an imaging optics chamber separated from the sample chamber and enclosing the imaging optics.

The examination system configured for microscopic examination further provides an incubation mode in which the sample chamber is incubated by supply of an incubation atmosphere generated by said incubation environment conditioning unit. This can comprise supplying specific gases or humidity into the sample chamber and heating the sample chamber. Said sample chamber might be a stage top chamber or a cage chamber as mentioned above. Preferably, said microscope is configured to provide both, a stage top chamber and a cage chamber, one of which a user can select to use when examining the sample. Further details of the specific kind of sample chambers will be provided later.

The inventors have recognized that setting and adjusting temperatures and other parameters of the incubator and the microscope is difficult and time consuming. In a perfectly balanced system, all components of the microscope surrounding the sample as well as the sample itself should be at a mutual setpoint of the temperature. That means the sample itself, the sample container, the objective and the air temperature above and below the sample plane need to be close to each other. Usually, therefore, a cage incubator also includes the objective and the objective revolver as well as the sample bottom side in the incubated volume as mentioned above. In addition, especially for use with a stage top incubator, there are multiple devices available to introduce and to control the exact temperature of the objective itself (e.g., silicone pads with integrated heating foils, wrapped around the objectives).

A cage incubator usually includes the components and the volume beneath the sample plane inside the incubated volume. However, this kind of setup has some drawbacks, as the inventors have recognized; it is (a) a very poorly ventilated area inside the incubator assembly with (b) no sensor to check temperatures around the objectives and (c) with no ability to change the air temperature in the substage compartment without changing the overall temperature setpoint of the system. These systems are fully system integrated and therefore (d) cannot compensate for introduction of warmth into the substage volume by electronic components of the microscope during operation or varying operation modes of the microscope.

A heating collar could be attached to the objectives itself; this can help to, partially, overcome the problem (c) mentioned before. However, such collar cannot be mounted to all types of objectives (e.g. motorized objectives) and it is solely under the control of the incubator, but not of the microscope. Air surrounding the microscope cannot be controlled with such a solution.

While in existing configurations, the microscope and the incubator are two standalone systems, the incubator may display current readouts in the microscope user interface and may receive setpoint commands from the microscope software, but each system is operating and controlling independently.

The controlling system of an embodiment of the invention, however, is configured to performing specific steps in order to operate the examination system. These steps comprise: receiving a target setpoint of at least one examination parameter like a temperature of the sample, upon user input via said user interface; and selecting, based on the received at least one target setpoint, predefined adjustment setpoints for at least one incubation environment parameter of the incubation mode like a temperature in the sample chamber or humidity and for at least one microscope parameter.

These predefined adjustment setpoints are preferably part of one or more datasets; for example, a dataset can have the form of a look-up table comprising target setpoints of the examination parameter being correlated to specific adjustment setpoints of at least one of the incubation environment and microscope parameters. Such specific correlations might be obtained from tests and/or simulations and, preferably, represent optimum examination conditions for the sample. Different datasets or look-up tables can be used for different incubation environment and/or microscope parameters. In other words, the adjustment setpoints for the incubation environment parameters and the microscope parameters are selected from one or more datasets, each dataset comprising adjustment setpoints of one or more of at least one of the incubation environment parameters and the microscope parameters, correlated to target setpoints of the sample temperature.

Further, the controlling system is configured to operating the incubation environment conditioning unit and the microscope based on the selected (predefined) adjustment setpoints. This, for example, comprises operating a heater and a pump in the incubation environment conditioning unit and a fan in the microscope such that the selected adjustment setpoints are reached; this typically requires some time after starting the operation upon the user input and requires measurement of current values of these parameters and feedback for closed-loop control.

An idea of an embodiment of the present invention, thus, is to integrate a microscope and incubation solution by implementing feedback control between the two systems. One click for the user in the user interface (this might be correlated with a microscope software), setting, for example, the desired temperature of the sample, can change multiple parameters in parallel in the incubator and in the microscope. Thereby, both systems work together synergistically to bring the entire examination system with objective, sample, sample chamber and substage volume (imaging optic chamber) temperatures to a perfect balance.

In particular, changing the target setpoint for the temperature of the sample by the user will force the incubator and the microscope to make use of various parameter datasets or look-up tables, which contain predefined adjustment setpoints of parameters, which preferably have been verified by integration testing before.

As mentioned above, a preferred examination parameter for which the user can set the target setpoint, is the temperature of the sample. Nevertheless, target setpoints for one or more other or additional examination parameters can, preferably, be set via user input and the user interface. These parameters can include carbon dioxide concentration in the sample chamber, and oxygen concentration in the sample chamber. Depending on the specific sample and/or the specific examination procedure to be performed, target setpoints for such examination parameters can be used (if provided by the incubation environment conditioning unit). Note that a further parameter of particular relevance for such sample examination can be (relative) humidity (concentration of water in the air or atmosphere) in the sample chamber. Choosing a target setpoint for this parameter is not an easy task for the user since there is a strong dependence on other parameters, which can result in too high humidity and cause condensation or evaporation of the sample in the chamber... Therefore, in a preferred embodiment of the invention, , the appropriate humidity as an incubation environment parameter is automatically provided by the incubation environment conditioning unit in dependency on an (adjustment) setpoint or value of one or more of the temperatures, which has already been selected by the user or from the dataset). Nevertheless, switching on and off humidity can be provided, i.e., a user can choose whether or not to provide controlled levels of relative humidity in the sample chamber at all.

Preferably, the at least one incubation environment parameter comprises at least one of the group of temperature in the sample chamber, and humidity in the sample chamber. The temperature in the sample chamber, in particular, relates to the temperature of the air or atmosphere in the sample chamber. Further, the incubation environment carbon dioxide concentration in the sample chamber, and/or oxygen concentration in the sample chamber can be used. Note that the latter ones can be used either as examination parameters or as incubation environment parameters.

As mentioned earlier, also an adjustment setpoint for at least one microscope parameter is selected. Such at least one microscope parameter comprises, in particular, an operation speed of a fan of the microscope, which fan is configured to ventilate the imaging optics chamber. Note that operation speed of the fan can also include no operation of the fan (speed zero). Depending on the fan, there might be only the choice between zero and a specific operation speed; or the operation speed may have different values to be set or may be set continuously within a certain range. Alternatively or additionally, such microscope parameters can comprise at least one of the group of a temperature in the imaging optics chamber (i.e., the temperature of the air or atmosphere in the imaging optics chamber), and a temperature of the imaging optics. Such fan typically influences both temperatures. These temperatures, in turn, have influence on the sample temperature, in particular, if the sample stage is arranged near the imaging optics or an objective being heated from electronics. A further microscope parameter can be the sample temperature; it is correlated to the temperature of an immersion medium, if used, and the temperature of the immersion medium has an effect on the diffraction index. This diffraction index, in turn, might be used to adapt the imaging optics.

To sum up, these adjustment setpoints include, in particular, setpoints for incubator ambient air temperature in cage or stage top mode, as well as dedicated humidity setpoint values. The later values, in particular, ensure that at every temperature setpoint, neither condensation nor evaporation of the sample or inside the sample chamber occurs. This is, why humidity should not be used as an examination parameter for which the user can choose the target setpoint, but humidity shall be set automatically according to predefined adjustment setpoints or values as mentioned. A separate dataset or look-up table can be used for the temperature in the sample chamber and for the humidity.

In addition, a further dataset or look-up table can ensure variable use of the heat generated by the microscope electronic components to push the temperature of the objective and the substage volume (in the imaging optics chamber) close to the temperature of the sample. Further, such datasets can also be of other types than look-up tables; for example, characteristic lines, curves, or functions can be used

For example, when zebra fish (typical target setpoint for the temperature in the sample: 28°C) or yeast (30°C) are imaged with the examination system, the fan or fan assembly introduces air from the surrounding room into the imaging optics chamber and warm air is sucked out of the system. The electronic heat is thereby drained from the objectives and the objectives equilibrate quite close to the sample temperatures. This procedure is applied, for example, from room temperature up to a verified temperature limit in the sample that assures that the temperature of the objective, the air temperature above and below the stage as well as the sample temperature show deviations as small as possible.. Thereby, the objective temperature closely follows the sample temperature in this range.

However, if performing experiments with the examination system at temperatures higher than the aforementioned temperature limit, such as cell culture experiments (at 37°C, for example), operation of the fan or fan assembly is stopped, allowing the electronic components to introduce additional heat to the objective. This again pushes the temperature of the objectives close to the higher temperature of the sample. This procedure is applied, for example, between room temperature and 45 °C.

Advantageously, the controlling system is further configured to: determining when at least one of: the at least one examination parameter, the at least one incubation environment parameter, and the at least one microscope parameter have reached values within a predetermined range around the respective selected adjustment setpoint or have reached the value of the selected adjustment setpoint; and indicating to the user, via said user interface, that the examination system is ready for examination. Indicating to the user can comprise displaying corresponding text on a display, activating a (real or virtual) lamp (like switching from red to green) or the like. In addition, playing a sound is an option. This allows a user to do anything else while waiting for the examination system to be ready for use and examination of the sample.

A further preferred way of operating the incubation environment conditioning unit and the microscope based on the selected adjustment setpoints is when the controlling system is configured to determining whether at least one of the temperature of the sample, the temperature in the sample chamber, the temperature in the imaging optics chamber, and the temperature of the imaging optics have reached a respective predefined value. The respective predefined value can be either the value defined by the respective target setpoint or selected adjustment setpoint, or a value within a range around the respective target setpoint or selected adjustment setpoint (in the sense of a threshold).

Note that each of the mentioned temperatures is either an examination parameter, an incubation environment parameter or a microscope parameter. Nevertheless, each of these temperatures has impact on the temperature of the sample and in the sample chamber and, thus, what the maximum value of humidity (or concentration of water in the atmosphere) in the sample chamber is. The higher the temperature in the sample chamber, for example, the higher the maximum value (saturation) of humidity (or relative humidity) is. In addition, said temperatures typically influence evaporation of water in the sample.

Thus, operating the incubation environment conditioning unit to control the humidity in the sample chamber is started after the respective temperatures have reached the respective predetermined values. Thus, condensation of water at the sample or anywhere else in the sample chamber at starting time, when the temperatures are low, can be prevented. In addition, undesired evaporation of water can be prevented. It is not necessary to wait with controlling humidity until the target or selected adjustment setpoints have been reached; rather, controlling humidity might be started before that because increasing humidity also will take some time. The specific range around the setpoints (threshold) may be determined individually for individual situations.

As mentioned above, the examination system combines two sub systems, an incubation environment conditioning unit and a microscope. Typically, each of these two sub systems has its own control unit or the like. In a preferred embodiment, the controlling system comprises a microscope control unit (typically, comprising a processor) configured to operating the microscope based on the selected adjustment setpoints for the microscope parameters, and at least one incubation control unit (typically, comprising a processor) configured to supplying the incubation atmosphere based on the selected adjustment setpoints for the incubation environment parameters. This means that both control units together form the controlling system and, basically, allows each control unit to take care of its respective parameters, including receiving the target setpoint and selecting the respective adjustment parameters. For example, the microscope control unit comprises at least one dataset for the microscope parameters with respective adjustment setpoints, and the incubation control unit comprises at least one dataset for the incubation environment parameters with respective adjustment setpoints. Thus, in a preferred embodiment, selecting the predefined adjustment setpoints for the incubation environment parameters can take place in the incubation control unit and selecting the predefined adjustment setpoints for the microscope parameters can take place in the microscope control unit Receiving the target setpoint of the at least one examination parameter from said user interface can take place in the microscope control unit (or also in the incubation environment control unit).

However, in another preferred embodiment, said microscope control unit is further configured to: receiving the target setpoint of the at least one examination parameter from said user interface; selecting the predefined adjustment setpoints for the incubation environment parameters and the microscope parameters; and transmitting the selected adjustment setpoints for the incubation environment parameters to said incubation control unit. Thus, the microscope control unit is used for operating or running any necessary software for dealing with the user input and selecting the appropriate adjustment setpoints for all parameters, based on the datasets. This also means that the datasets are, preferably, provided at the microscope control unit, e.g., in a memory of the control unit The incubation control unit, thus, only receives relevant setpoints for the incubation environment parameters and does not have to deal with any specific determining or selection processes. Note that the at least one incubation control unit does not necessarily need to be part of the controlling system.

The examination system is, preferably, configured to displaying to a user, via said user interface, current values of the at least one examination parameter like the sample temperature, and of the at least one incubation environment parameter. This provides an overview of the current process situation to the user. In addition, a current value of the microscope parameter (e.g., fan on or off) can be displayed, if required.

Advantageously, the examination system further comprises an atmosphere regulation module for controlling the at least one incubation environment parameter in the incubation mode. Such atmosphere regulation module can be connected to the controlling system or the incubation control unit This allows further regulating or fine-tuning of the incubation atmosphere. To this end, sensors may be provided in the atmosphere regulation module or at least sensor signals may be provided to the atmosphere regulation module, the sensors signals representing values of the at least one incubation environment parameter. If an actual value of such a parameter deviates from the setpoint of this parameter, the atmosphere regulation module can regulate/readjustthe respective parameter.

To this purpose, it is advantageous if the atmosphere regulation module is in communication with the incubation control unit and/or a pump control unit for the incubation mode. In this embodiment, if the temperature or humidity of the incubation atmosphere deviates from the corresponding setpoints, the atmosphere regulation module can send a request to the corresponding (pump) control unit for regulating/readjusting the temperature or humidity to the correct setpoints. On the other hand, the atmosphere regulation module can be provided with corresponding means for such a regulation/readjustment. For example, the atmosphere regulation module may comprise at least one of a heater/cooler, and a humidifier for regulating temperature and/or humidity of the incubation atmosphere on its own without communication with the corresponding (pump) control unit, once the adjustment setpoint has been provided.

As already pointed out, in a preferred embodiment, the examination system is configured to providing or supplying incubation atmosphere to two different kind of sample chambers. To this end, said microscope further comprises a microscope housing enclosing the illumination optics, the microscope stage and the imaging optics. Further, said microscope comprises an integrated sample chamber located within the microscope housing and formed by a separated housing section within said microscope housing. The imaging optics chamber is located within said microscope housing and separated from the sample chamber. Thus, a first type of sample chamber is a sample chamber integrated into the microscope housing. This type of sample chamber is also called a cage chamber like in a cage incubator providing a sample chamber.

A second type of sample chamber is one to be placed within said integrated sample chamber. This second type of sample chamber is also called a stage top chamber like in stage top incubator providing a sample chamber. Further, said housing section comprises said microscope interface configured for connection of the incubation environment conditioning unit to the integrated sample chamber and/or the stage top sample chamber to be placed within said integrated sample chamber. Note that such second type of sample chamber typically, is not fixed to the microscope or the integrated sample chamber, but can be placed therein upon requirements.

In such configuration, the examination system provides an individual incubation mode for each sample chamber. In particular, the examination system provides a first incubation mode and a second incubation mode, wherein, in the first incubation mode, the integrated sample chamber is incubated by supply of a first incubation atmosphere by said incubation environment conditioning unit, and, in the second incubation mode, the stage top chamber is incubated by supply of a second incubation atmosphere by said incubation environment conditioning unit Further, the controlling system is configured to: operating the incubation environment conditioning unit and the microscope based on the selected adjustment setpoints for a selected one of the first incubation mode and the second incubation mode. Note that this may require different datasets for the two incubation modes, which datasets correlate adjustment setpoints of one or more incubation environment parameters and/or microscope parameters correlated to target setpoints of the at least one examination parameter.

This provides a customer with two incubation solutions realised in the same examination system. In the first incubation mode, the incubated volume is reduced to the volume of the separated housing section within the microscope housing, which is designed specifically to the needs of the respective microscope and intended applications so that the inside volume is already decreased to a minimum.. The sample chamber allows for sufficient space to perform all necessary sample manipulations requested by the application and leaves enough space for additional necessary equipment Still the system enables a user to make use of a stage top incubator without changing to another system. This preferred embodiment combines a stage top and a sample chamber incubator in one single system, shares common components, and realizes reproducible incubation conditions with respect to temperature homogeneity, heat up phases, etc. For both types of sample chambers or incubation modes, the one-click solution for setting different parameters is available.

In a further embodiment of the invention, a method for microscopic examination of a sample by means of an examination system as explained above is provided. Such method comprises the following steps: providing a sample in the sample chamber (depending on the kind of examination system, this may include choosing one two different sample chambers); providing the target setpoint of the at least one examination parameter, upon user input via said user interface; and examining the sample by means of the microscope after defined threshold values of the predefined adjustment setpoints for the at least one incubation environment parameter of the incubation mode and for the at least one microscope parameter have been reached. These defined threshold values can correspond to the values as set with the adjustment setpoints or may differ slightly. In this regard, it is also referred to the remarks above, dealing with indicating when the examination system is ready for examination.

The adjustment setpoints for the incubation environment parameters and the microscope parameters, in particular, are selected from one or more datasets, each dataset comprising adjustment setpoints of one or more incubation environment parameters and/or microscope parameters correlated to target setpoints of the at least one examination parameter.

In a further embodiment of the invention, a method of operating an examination system configured for microscopic examination of a sample is provided; the examination system comprising a microscope, an incubation environment conditioning unit connected to said microscope, and a user interface. Said microscope comprises: an illumination optics, a microscope stage, an imaging optics, a sample chamber configured to receive the sample, a microscope interface configured for connection of the incubation environment conditioning unit to the sample chamber, and an imaging optics chamber separated from the sample chamber and enclosing the imaging optics. Further, said examination system provides an incubation mode in which the sample chamber is incubated by supply of an incubation atmosphere generated by said incubation environment conditioning unit.

The method comprises the following steps: receiving a target setpoint of at least one examination parameter, preferably comprising at least a target temperature of the sample, upon user input via said user interface; selecting, based on the received target setpoint of the at least one examination parameter, predefined adjustment setpoints for at least one incubation environment parameter of the incubation mode and for at least one microscope parameter; and operating the incubation environment conditioning unit and the microscope based on the selected adjustment setpoints. For further preferred embodiments of the examination system, it is referred to the remarks above, which apply here correspondingly.

For any further details, preferred embodiments and advantage of the methods, it is also referred to the remarks above, which include also method aspects and apply here correspondingly.

The invention also relates to a computer program with a program code for performing a method according to the invention when the computer program is run on one or more processors or on a controlling system according to the invention.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short description of the Figures

- Fig. 1a: schematically shows a perspective view of an examination system according to a preferred embodiment of the invention;
- Fig. 1b: schematically shows an examination system according to a further preferred embodiment of the invention;
- Fig. 2: schematically shows an examination system according to a further preferred embodiment of the invention and a flow diagram, for explaining different steps of operation;
- Figs. 3a and 3b: schematically show different steps of operating an examination system according to a further preferred embodiment of the invention;
- Figs. 4a and 4b: schematically show an examination system according to a further preferred embodiment of the invention in two different operating states.

### Detailed Description

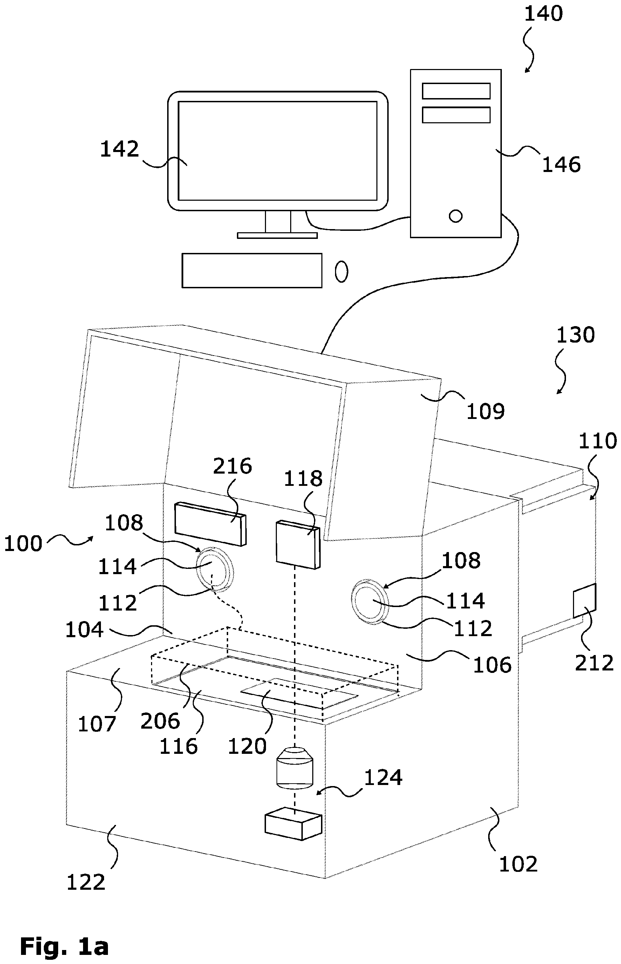

Fig. 1a schematically illustrates, in a perspective view, an examination system 130 for microscopic examination of a sample 120 according to a preferred embodiment of the invention. The examination system 130 comprises a microscope 100, an incubation environment conditioning unit 110, a controlling system 140 comprising, for example, a PC 146, and a user interface; the user interface 142 comprises, e.g., a display or display screen, keyboard and computer mouse for providing the user interface to a user.

The controlling system 140 comprises a microscope control unit 214, an incubation control unit 212 and, for example, a PC 146; the PC is connected with the user interface. Note that the functionality of the PC can also be integrated into the microscope control unit 214, for example. The user interface means can, alternatively, comprise a touch screen, for example.

The microscope 100 is used for microscopic examination of the sample 120 arranged on a microscope stage 116. A microscope housing 102 encloses an illumination optics 118, the microscope stage 116 and an imaging optics 124. An integrated sample chamber 106 is located within the microscope housing 102 and formed by a separated housing section 104 within said microscope housing 102. The housing section 104 comprises a hinged lid 109, which provides direct access to the microscope stage 116 for placing the sample 120 in the sample chamber 106 onto the microscope stage 116 and for exchanging samples 120 and/or manipulating samples 120 when the lid is opened.

The embodiment shown in Fig. 1a is an inverse transmitted-light microscope 100 where the transmitted-light illumination optics 118 is arranged within the housing section 104, while the imaging optics 124 is located below the microscope stage 116 in a second housing section, forming an imaging optics chamber 122. The imaging optics 124 typically includes a microscope objective and an image detector as the main components. The image detector usually comprises a camera, which generates microscopic images, which are typically displayed on the display screen of user interface 142 outside the microscope housing 102.

The construction of the microscope housing section 104 allows - after closing the lid 109 - to form a dedicated sample chamber 106, which constitutes a preferably sealed space which can be incubated such that during microscopic examination/imaging of living samples 120 like cells, the sample can be kept under favorable and stress-free environmental conditions. To this end, the housing section 104 comprises an interface 108 for connection of the external incubation environment conditioning unit 110 to the sample chamber 106. The interface 108 is configured to provide a connection between the incubation conditioning unit 110 and the sample chamber 106, such that environmental conditions in the sample chamber 106 can be controlled when the conditioning unit 110 is connected to the interface 108. This configuration implements the first incubation mode ("sample chamber incubation").

In the embodiment shown, the interface 108 as a part of the housing section 104 comprises two openings 112 in the backside of the housing section 104, each opening 112 being configured to receive a conduit 114. Incubation atmosphere can be introduced through at least one of the conduits 114 into the sample chamber 106. Depending on the leakage of the sample chamber 106, a part of the incubation atmosphere is allowed to escape the sample chamber 106. On the other hand, a part of the incubation atmosphere can be withdrawn from the sample chamber 106 via another one of the conduits 114.

Suitable incubation atmospheres comprise, e.g., air with a predefined content of H₂O (water or water vapor, relative humidity) and a predefined content of CO₂ (carbon dioxide). It is also desirable to conduct hypoxia experiments with a deficiency of oxygen in the atmosphere. For further details about specific parameters and suitable or preferred ranges, please refer to the description of the following Figs.

Note that controlling the relevant parameters (incubation environment parameters like temperature of the sample chamber, humidity, carbon dioxide concentration, oxygen concentration; microscope parameters like fan speed) will be performed automatedly by means of the controlling system 140, with, for example, a target setpoint for the temperature of the sample as an examination parameter being set by a user.

In order to control the above parameters, it is preferred to arrange sensors in or at at least one of the conduits 114, of the sample chamber 106, and of the microscope stage 116 close to the sample 120. In a preferred embodiment, at least some of the sensors are integrated into a conduit 114 for supplying incubation atmosphere to the sample chamber 106.

As shown in Fig. 1a, the housing section 104 is bounded downward by a working surface 107 including an upper side of the microscope stage, in other words the microscope stage tabletop. This construction provides a user-friendly access to the working area for placing and manipulating the sample 120. To the other sides, the housing section is bounded by the inner sides of the lid 109 and the backside of the housing section 104 itself.

For increasing the live span of the imaging optics 124 and in case of using immersion objectives, for example, air condition and/or temperature-control for the imaging optics chamber 122 is provided. To this end, a fan 126 for ventilating the imaging optics chamber 122 is provided, which can be operated or controlled by the controlling system 140 or, in particular, a the microscope control unit 214 as a part thereof.

Said microscope control unit 214 further controls functional components like the illumination optics 118, the microscope stage 116 and the imaging optics 124 of the microscope 100 for controlling the microscopic examination/imaging of the sample 120. Typically, the user interface 142 provides graphical user interface (GUI) displayed on the display screen for a user-friendly operation of the microscope 100.

The controlling system 140 is further configured to control the operation of the incubation environment conditioning unit 110 when the conditioning unit 110 is connected to the interface 108, in particular, via the incubation control unit 212 provided for such purposes. Cables and lines for communication between the PC 146 and/or the microscope control unit 214 and the incubation control unit 212 can be guided through one or more of the above openings/conduits of the interface 108.

As shown in Fig. 1a, the default configuration is the first incubation mode, namely, in this embodiment, the sample chamber incubation mode. The incubation environment conditioning unit 110 comprises an incubation control unit 212, which is shown in Fig. 1a. Within the housing section 104, at its backside, there is an atmosphere regulation module interface to which an atmosphere regulation module 216 is connected to as shown in Fig. 1a.

The above-mentioned sensors for detecting one or more of the incubation atmosphere parameters are preferably arranged in the sample chamber 106, particularly in or at the atmosphere regulation module 216. The atmosphere regulation module 216 is connected to the incubation environment conditioning unit 110 via a communication line. In that way, depending on the sensor signals, which correspond to the parameter values, the first atmosphere regulation module 216 together with the incubation control unit 212 can provide a feedback control to set the first group of parameters of the first incubation atmosphere to a desired setpoint. This process will be further described in connection with the following Figs.

In addition, a stage top sample chamber 206 is indicated by dashed lines. Such stage top (or tabletop) sample chamber is to be placed within the integrated sample chamber 106; a connection to an opening 112 and/or a conduit 114 can also be established in order to provide the respective incubation environment in that smaller stage top sample chamber 206. The user can select which sample chamber to use, wherein using the tabletop sample chamber 206 requires placing it in the integrated sample chamber 106.

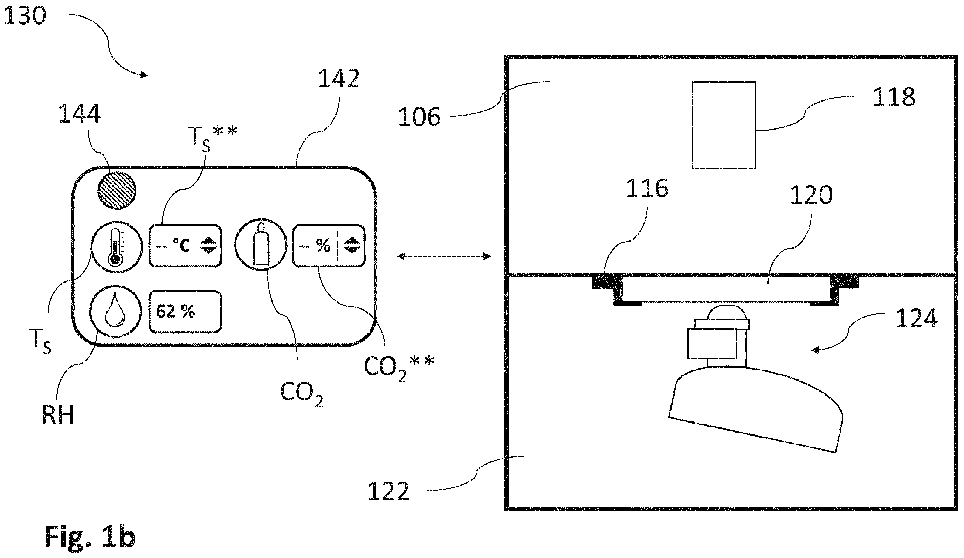

Fig. 1b schematically illustrates an examination system 130 according to the invention in a further preferred embodiment The examination system 130 can correspond to the one shown in Fig. 1a. However, Fig. 1b, in particular, illustrates the components of the examination system 130 and the parameters, which are of particular relevance within an embodiment of the present invention. Note that like reference numerals will be used for like components and parameters throughout the Figs.

The left side of Fig. 1b illustrates the user interface 142; the user interface 142 displays a symbol for temperature Ts of the sample as an examination parameter and, next to it, a field for input of a target setpoint T_{S}** for this temperature of the sample. The user can, for example, rise and lower the target setpoint for that temperature via clicking on respective soft buttons. Further, the user interface 142 displays a symbol for temperature carbon dioxide concentration CO₂ in the sample chamber as an examination parameter and, next to it, a field for input of a target setpoint CO₂** for this concentration. The user can, for example, rise and lower the target setpoint for that concentration via clicking on respective soft buttons. Note that, typically, the most relevant examination parameter is the temperature of the sample, while carbon dioxide concentration change or use might be disabled. In the following, the carbon dioxide concentration will not be considered (or be considered as disabled).

Further, the user interface 142 displays a symbol for (relative) humidity RH in the sample chamber. Humidity is not an examination parameter but an incubation parameter, which is automatedly set. The user is not able to change any setpoint for it. Thus, only a current value might be displayed; also, an option might be to allow switching on and off the use of humidity (water in the sample chamber) at all. Further, the user interface 142 displays a colored symbol 144, representing a (virtual) lamp. Such lamp might switch from red to green when the examination can start, for example. This will be explained in more detail later.

The right side of Fig. 1b illustrates the sample chamber 106 and the imaging optics chamber 122 in a very schematic way. In particular, the illumination optics 118, the microscope stage 116, the sample 120 and the imaging optics 124 are shown. Note that the imaging optics, by means of example, comprises a motorized objective on a turret

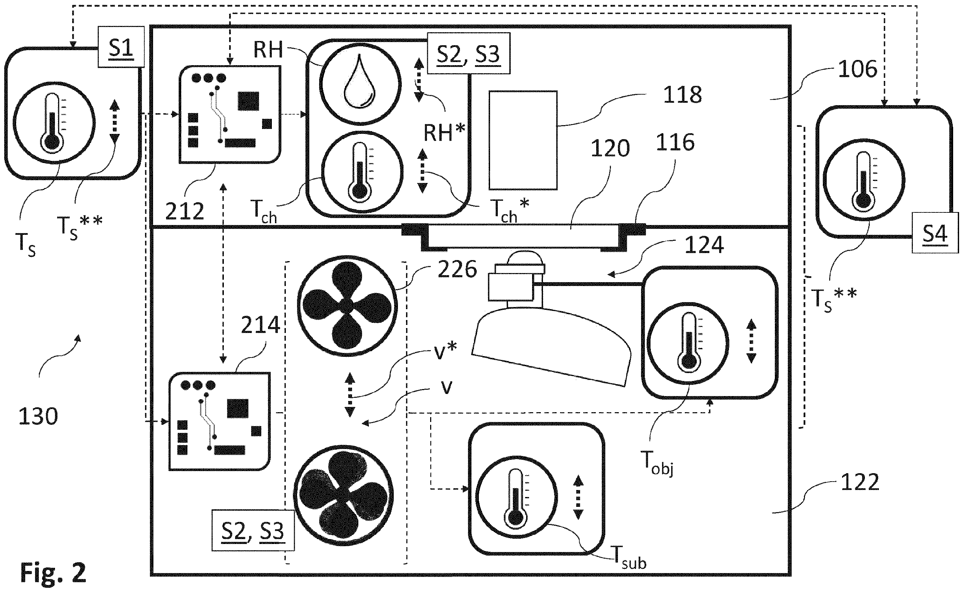

Fig. 2 illustrates an examination system 130 according a further preferred embodiment of the invention and a flow diagram, based on which different steps of operating the examining system will be explained. In particular, Fig. 2 shows the sample chamber 106, the imaging optics chamber 122, the illumination optics 118, the microscope stage 116, the sample 120 and the imaging optics 124 similar to Fig. 1b. In addition, the incubation control unit 216 and the microscope control unit 214, both of which might be part of the controlling system, are schematically illustrated.

In a first step, S1, a user can set a target setpoint T_{S}** for the temperature Ts of the sample via the user interface, as described with respect to Fig. 1b above. The controlling system and, in particular, the microscope control unit 214 and/or the incubation control unit 216, will then receive this target setpoint T_{S}**.

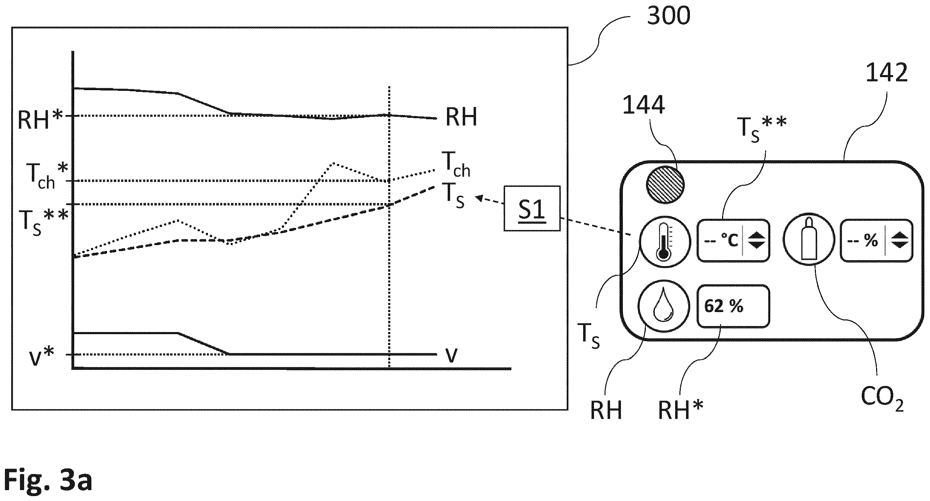

In a further step, S2, the controlling system selects, based on the received target setpoint T_{S}**, a predefined adjustment setpoint RH* for the (relative) humidity RH in the sample chamber, and a predefined adjustment setpoint T_{ch}* for the temperature T_{ch} of the sample chamber. These two parameters are incubation environment parameters as they refer to the sample chamber 106, in which the incubation environment is provided, Further, a predefined adjustment setpoint v* for the operating speed v of the fan 226 in the imaging optics chamber is selected. This parameter is a microscope parameter as it refers to components of the microscope and the corresponding imaging optics chamber 122, in which the fan is arranged. The way of how to select such adjustment setpoints based on the target setpoint will be described below with respect to Fig. 3a.

Then, in a further step, S3, the incubation environment conditioning unit 110 and the microscope 100 are operated, by means of the controlling system or the microscope control unit 214 and/or the incubation control unit 216, respectively, such that the three parameters RH, T_{ch} and v will arrive at the respective adjustment setpoints RH*, T_{ch}* and v* selected before.

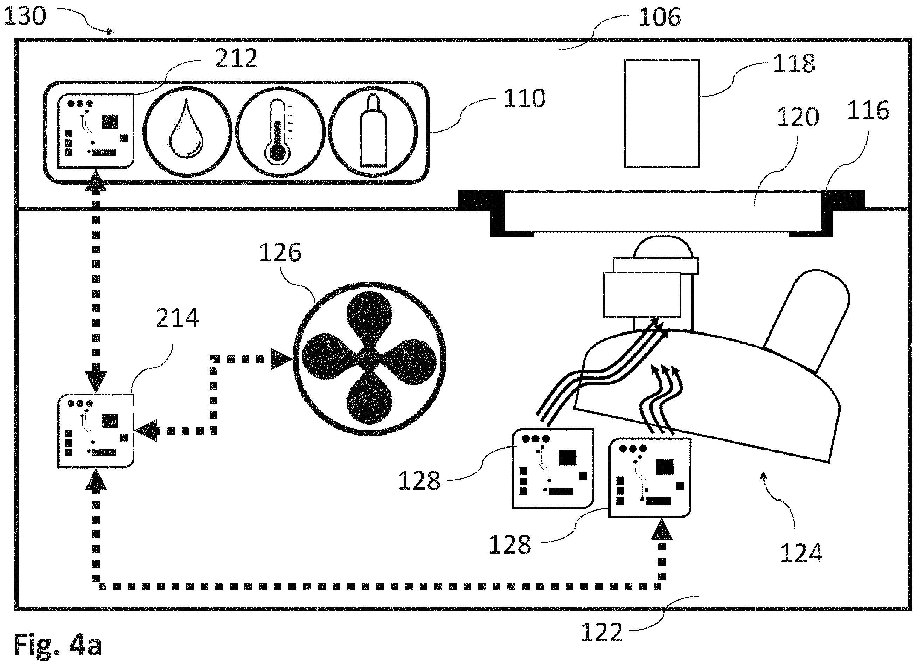

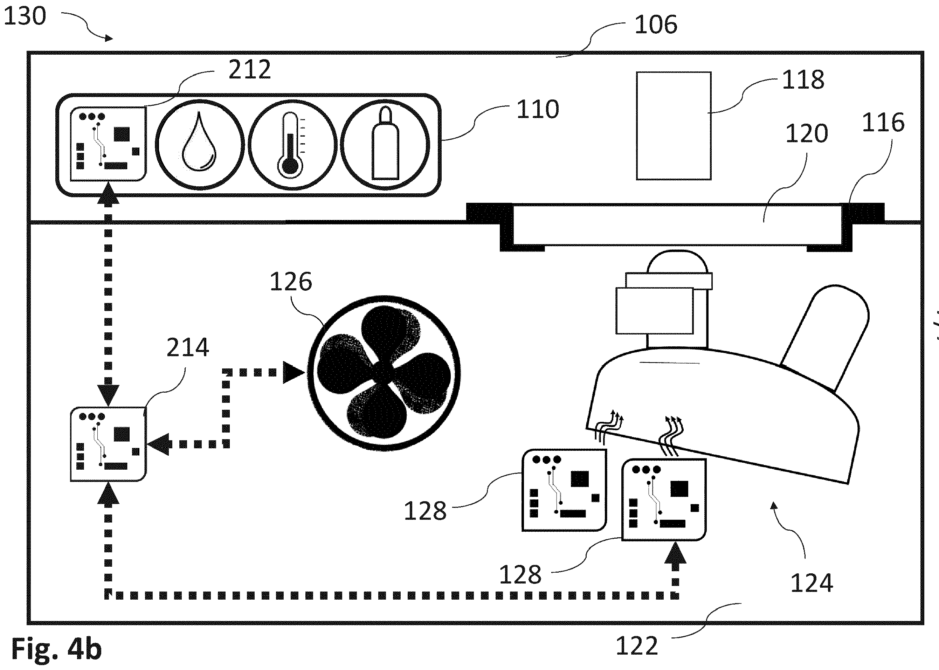

In the imaging optics chamber 122, this means that a temperature T_{sub} in the imaging optics chamber and a temperature T_{obj} of the imaging optics (comprising an objective) will change due to heat generated by operation of the imaging optics (including, e.g., an imaging detector and a motor for the motorized objective and other electronic components). Depending on the operating speed v of the fan, the heat generated by the electronic components will have more or less impact; please refer to Figs. 4a, 4b for a more detailed explanation. As mentioned above, also the temperatures T_{sub} and T_{obj} (and the sample temperature Ts) could be used as microscope parameters with respective adjustment setpoints.

Note that controlling these parameters in order to achieve the respective adjustment setpoint, typically comprises measuring (or in another way determining) the current values of these parameters repeatedly, feed these current values back to the controlling system, and changing actuating variables, if necessary. Note that this can be performed by means of typical closed-loop control. The atmosphere regulation module 216 and the sensors mentioned above might be used in this regard.

After some time, the adjustment setpoints will have been reached and, thus, also the target setpoint T_{S}** for the temperature of the sample will have been reached, as shown with step S5.

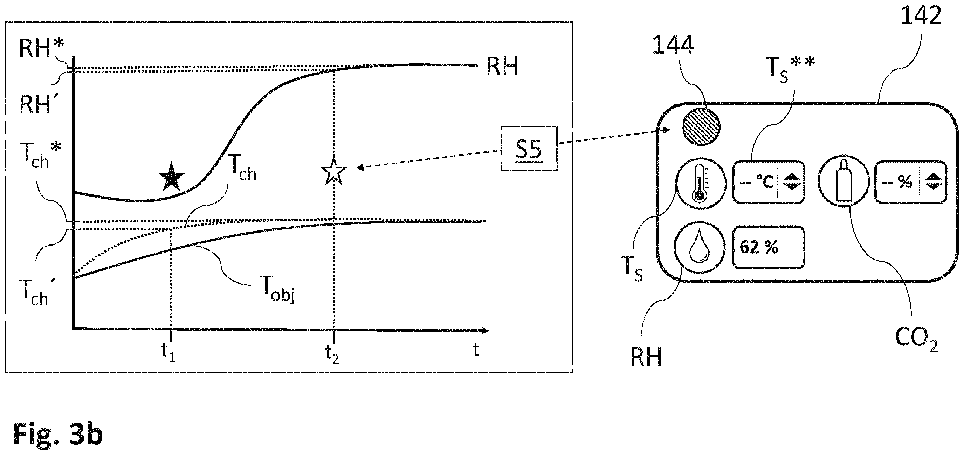

Figs. 3a and 3b schematically illustrate different steps of operating an examination system according to a further preferred embodiment of the invention, in particular, as described with respect to Fig. 2. In addition, the user interface 142 displaying all the elements as shown in Fig. 1 is illustrated on the right side of each of Figs. 3a and 3b.

The left side of Fig. 3a illustrates a dataset 300, in which adjustment setpoints for temperature T_{ch} of the sample chamber, humidity RH in the sample chamber (incubation environment parameters) and operation speed v of the fan (microscope parameter) are correlated to target setpoints of the temperature Ts of the sample (examination parameter). In particular, graphical lines illustrate the dataset 300 for each of these parameters, where values of the parameters are defined by the vertical axis. Note that the specific values or courses of the lines are not relevant for explanation of the invention. Physical units of the parameters might be °C (degrees Celsius) for the temperatures, % (percentage) for the humidity, and rpm (rotations per minute) for the operating speed of the fan (the latter might, a simple example, also be expressed by on/off).

Next, the way of how to select adjustment setpoints based on a target setpoint will be described. As mentioned above, a user can choose a desired temperature of the sample as target setpoint T_{S}**. In the specific dataset 300 shown, the corresponding adjustment setpoints RH*, T_{ch}* and v* are found by drawing a vertical line through the point in which the line of Ts crosses the target setpoint T_{S}**. The adjustment setpoints RH*, T_{ch}* and v* found in this way will then be selected, and the system is operated based on them.

Note that the graphical illustration of dataset 300 is mainly a preferred way to illustrate the correlations between the different parameters and their setpoints. A typical way of how to implement such dataset in a controlling system can be, for example, as a plurality of sets of values, each set comprising one value for each parameter. This allows, for example, choosing the set in which the value of the temperature of the sample matches the given target setpoint.

The left side of Fig. 3b illustrates, for a preferred embodiment of the invention, a change of the humidity RH in the sample chamber, of the temperature T_{ch} in the sample chamber, and of the temperature T_{obj} of the objective over time t With starting operation of the incubation environment conditioning unit and the microscope (see left end of the diagram), the temperature T_{ch} in the sample chamber, and the temperature T_{obj} of the objective rise over time and arrive at their final values; this final value corresponds, for the temperature T_{ch} in the sample chamber, to the selected adjustment setpoint T_{ch}*. Note that the final value of the temperature T_{obj} of the objective is very close to the one of the temperature T_{ch} in the sample chamber, because the objective is very close to the sample 120 and the sample chamber 106.

As can be seen in the diagram, the humidity RH decreases with the start of operation. The reason is that changing or controlling humidity RH is not started together with the other parameters. Rather, humidity is not changed actively at the beginning. However, due to the increase of the temperature T_{ch} in the sample chamber, humidity decreases slightly. The reason as to why humidity is not actively increased from the beginning is that water might condensate at the sample or other components in the sample chamber if the humidity was increased too high with low temperatures around. At low temperatures, maximum relative humidity in air is lower than at higher temperatures.

At time t₁, also indicated with a filled star symbol, the temperature T_{ch} in the sample chamber has reached a (threshold) value T_{ch}', which is considered high enough that increasing humidity RH would not lead to condensation. Thus, the controlling system starts operating the incubation environment conditioning unit 110 such that humidity RH is increased in order to achieve the selected adjustment setpoint RH*.

At time t₂, also indicated with an empty star symbol, the temperature T_{ch} in the sample chamber has reached the selected adjustment setpoint T_{ch}*, and humidity RH has reached a value RH' slightly below the respective selected adjustment setpoint RH*. These conditions are, by means of example, considered sufficient for starting examination of the sample as the temperature of the sample has reached the target setpoint. This is indicated to the user via user interface 142 in that, for example, the symbol 144 (virtual lamp) changes its color from red to green.

Note that, depending on the circumstances, the time for indicating that the examination system is ready for examination, can also be set to a time at which also humidity RH has reached the selected adjustment setpoint. In addition, other parameters can be included.

Figs. 4a and 4b illustrate examination system 130 according to a further preferred embodiment of the invention in two different operating states. The examination systems 130 correspond to the one shown in Fig. 2. As mentioned with respect to step S2 in Fig. 2, the operating speed v of the fan - it is a microscope parameter - is set to the selected adjustment setpoint.

Fig. 4a illustrates a situation in which the operating speed is set to zero, i.e. the fan 128 is not operating; thus, no ventilation is used and any heat generated from electronic components 128 of the imaging optics 124 (or other heat sources in the imaging optics chamber 122) is not disturbed. This is indicated with long arrows starting at the components 128. Consequently, the imaging optics 124 and, in particular, the objective is heated and the temperature of the objective rises and reaches a relatively high value. Note that, typically, the objective is coupled thermally and optically to the microscope stage (sample carrier) via an immersion medium. As most of the heat for heating the sample is introduced via the incubation atmosphere, heat of the sample would flow to the objective (and imaging optics chamber) if the respective temperatures were too low. Thus, heat generated by the electronic components 128 is used to introduce heat to the objective and imaging optics chamber. The sample chamber is heated in parallel by the incubation unit. The examination system reaches a balanced state of operation where temperatures of the sample, of the objective and of the air below and above the sample plain are almost identical, in particular at higher temperatures. This is preferred for high target setpoints for the temperature of the sample, to conduct for example living cell culture experiments (typically about 37°C).

Fig. 4b illustrates a situation in which the operating speed is set to a specific value higher than zero, i.e. the fan 128 is operating; thus, the imaging optics chamber is ventilated and the electronic components 128 of the imaging optics 124 (or other heat sources in the imaging optics chamber 122) are actively cooled and, thus, less heat is generated; warm air is sucked outside the system. This is indicated with short arrows starting at the components 128. Consequently, the imaging optics chamber 122 and the imaging optics 124 and, in particular, the objective is less heated than with no fan operating and the temperature of the objective does not rise or, at least rise less than with no fan operation; it reaches a relatively low value and the objective does only introduce - if at all - a minimum of heat in the sample.. The sample chamber is heated in parallel only very little by the incubation unit. The examination system therefore reaches a balanced state of operation where temperature of the sample, the objective and the air temperature below and above the sample plain are almost identical at colder temperatures. This is preferred for low target setpoints for the temperature of the sample such as required for zebra fish (e.g., 28°C) and yeast (e.g., 30°C).

Note that, depending on the functionality of the fan, different operating speeds may be used for different target setpoints for the temperature of the sample. However, also a simple fan with only on and off mode can be used.

To sum up, an idea of an embodiment of the present invention is to integrate microscope and incubation solution by implementing feedback control between the two systems. It provides a one-click solution for the user and, e.g., the microscope software changes multiple parameters in parallel in the incubator and the microscope. Thereby, both systems work together synergistically to bring objective, sample, sample chamber and imaging optics chamber (substage volume) temperatures to a perfect balance. In particular, the user does not have to take care about any other than the examination parameters. In a preferred embodiment, the only value the user has to provide is the desired sample temperature. Any remaining parameters will automatically be set to optimal values.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1a to 4b. Alternatively, a microscope may be part of or connected to a system as described in connection with one or more of the Figs. 1a to 4b. Fig. 1a shows a schematic illustration of a system 130 configured to perform a method described herein. The system 130 comprises a microscope 100 and a computer (controlling) system 140. The microscope 100 is configured to take images and is connected to the computer system 140. The computer system 140 is configured to execute at least a part of a method described herein. The computer system 140 may be configured to execute a machine learning algorithm. The computer system 140 and microscope 100 may be separate entities but can also be integrated together in one common housing. The computer system 140 may be part of a central processing system of the microscope 100 and/or the computer system 140 may be part of a subcomponent of the microscope 100, such as a sensor, an actor, a camera or an illumination unit, etc. of the microscope 100.

The computer system 140 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 140 may comprise any circuit or combination of circuits. In one embodiment, the computer system 140 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit Other types of circuits that may be included in the computer system 140 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 140 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 140 may also include a display device, one or more speakers, and a keyboard and/or controller, which can include a mouse, trackball, touch screen, voice-recognition device, or any other device that permits a system user to input information into and receive information from the computer system 140.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100: microscope
- 102: microscope housing
- 104: separated housing section
- 106: sample chamber
- 107: working surface
- 108: microscope interface
- 109: lid
- 110: incubation environment conditioning unit
- 112: opening
- 114: conduit
- 116: microscope stage
- 118: illumination optics
- 120: sample
- 122: imaging optics chamber
- 124: imaging optics
- 126: fan
- 128: electronic components
- 130: examination system
- 140: controlling system
- 142: user interface
- 144: symbol on user interface
- 146: PC

- 206: stage top chamber
- 212: incubation control unit
- 214: microscope control unit
- 216: atmosphere regulation module

- Ts: temperature of the sample
- T_{ch}: temperature in the sample chamber
- T_{obj}: temperature of the objective
- T_{sub}: temperature in the imaging optics chamber
- RH: humidity in the sample chamber
- CO₂: carbon dioxide concentration

- T_{S}**, CO₂**: target setpoints
- T_{ch}*, RH*: adjustment setpoints
- T_{ch}', RH': values

- t₁, t₂: times

## Claims

1. A controlling system (140) for operating an examination system (130) configured for microscopic examination of a sample (120), the examination system (130) comprising a microscope (100), an incubation environment conditioning unit (110) connected to said microscope (100), and a user interface (142),
said microscope (100) comprising: an illumination optics (118), a microscope stage (116), an imaging optics (124), a sample chamber (106) configured to receive the sample (120), a microscope interface (108) configured for connection of the incubation environment conditioning unit (110) to the sample chamber (106), and an imaging optics chamber (122) separated from the sample chamber (106) and enclosing the imaging optics (124),
wherein the examination system (130) configured for microscopic examination provides an incubation mode in which the sample chamber (106) is incubated by supply of an incubation atmosphere generated by said incubation environment conditioning unit (110),
wherein the controlling system (140) is configured to:
receiving (S1) a target setpoint (T_{S}**, CO₂**) of at least one examination parameter (Ts, CO₂), upon user input via said user interface (142);
selecting (S2), based on the received at least one target setpoint (T_{S}**, CO₂**), predefined adjustment setpoints (T_{ch}*, RH*, v*) for at least one incubation environment parameter (T_{ch}, RH, CO₂) of the incubation mode and for at least one microscope parameter (v, T_{obj}, T_{sub}); and
operating (S3) the incubation environment conditioning unit (110) and the microscope (100) based on the selected adjustment setpoints.

2. The controlling system (140) of claim 1, wherein the target setpoint of the at least one examination parameter comprises at least a target temperature of the sample (T_{S}**).

3. The controlling system (140) of claim 1 or 2, wherein the at least one incubation environment parameter comprises at least one of the group of temperature (T_{ch}) in the sample chamber, and humidity (RH) in the sample chamber.

4. The controlling system (140) of claim 3, further configured to: automatedly providing a value for said humidity (RH) in the sample chamber, said value depending on a selected or chosen value of at least one of the temperature (Ts) of the sample, the temperature (T_{ch}) in the sample chamber, the temperature (T_{sub}) in the imaging optics chamber, and the temperature (T_{obj}) of the imaging optics.

5. The controlling system (140) of claim 3 or 4, wherein the at least one incubation environment parameter further comprises at least one of the group of a carbon dioxide (CO₂) concentration in the sample chamber, and an oxygen concentration in the sample chamber.

6. The controlling system (140) of any one of the preceding claims, wherein the at least one microscope parameter comprises at least one of the group of: an operation speed (v) of a fan (126) of the microscope (110), the fan (126) configured to ventilating the imaging optics chamber (122), a temperature (T_{sub}) in the imaging optics chamber, a sample temperature and a temperature (T_{obj}) of the imaging optics.

7. The controlling system (140) of claim 3, 4 or 5 and of claim 6 wherein the at least one incubation environment parameter comprises the humidity (RH) in the sample chamber, and wherein operating (S3) the incubation environment conditioning unit (110) and the microscope (100) based on the selected adjustment setpoints (T_{ch}*, RH*, v*) comprises:
determining whether at least one of the temperature (Ts)of the sample, the temperature (T_{ch}) in the sample chamber, the temperature (T_{sub}) in the imaging optics chamber, and the temperature (T_{obj}) of the imaging optics have reached a respective predefined value (T_{ch}'); and
starting operating the incubation environment conditioning unit (110) to control the humidity (RH) in the sample chamber after the respective temperatures have reached the predetermined values,
wherein the respective predefined value is the value defined by the respective target setpoint or selected adjustment setpoint, or a value (T_{ch}') within a range around the respective target setpoint or selected adjustment setpoint (T_{ch}*).

8. The controlling system (140) of any one of the preceding claims, further configured to:
determining when at least one of the group of: the at least one examination parameter, the at least one incubation environment parameter, and the at least one microscope parameter have reached values (RH') within a predetermined range around f the respective selected adjustment setpoint (RH*) or have reached the value of the selected adjustment setpoint (T_{ch}*); and
indicating (S5) to the user, via said user interface (142), that the examination system (130) is ready for examination.

9. The controlling system (140) of any one of the preceding claims, wherein the at least one examination parameter further comprises at least one of the group of a carbon dioxide concentration (CO₂) in the sample chamber, and an oxygen concentration in the sample chamber.

10. The controlling system (140) of any one of the preceding claims, wherein the adjustment setpoints (T_{S}**, RH*, T_{ch}*, v*) for the incubation environment parameters and the microscope parameters are selected from one or more datasets (300), each dataset comprising adjustment setpoints of one or more of the group of: the incubation environment parameters and the microscope parameters, correlated to target setpoints of the at least one examination parameter.

11. The controlling system of any one of the preceding claims, comprising at least one incubation control unit (212) configured to supplying the incubation atmosphere based on the selected adjustment setpoints (T_{ch}*, RH*) for the incubation environment parameters, and
a microscope control (214) unit configured to operating the microscope based on the selected adjustment setpoints (v*) for the microscope parameters.

12. The controlling system of claim 11, said microscope control unit (214) further configured to:
receiving the target setpoint (T_{S}**) of the at least one examination parameter (Ts) from said user interface (142);
selecting the predefined adjustment setpoints (T_{ch}*, RH*, v*) for the incubation environment parameters and the microscope parameters; and
transmitting the selected adjustment setpoints for the incubation environment parameters to said incubation control unit (212).

13. The controlling system (140) of any one of claims 1 to 10, comprising a microscope control unit (214) configured to operating the microscope (100) based on the selected adjustment setpoints (T_{ch}*, RH*) for the microscope parameters, said microscope control unit (214) further configured to:
receiving the target setpoint (T_{S}**) of the at least one examination parameter from said user interface (142);
selecting the predefined adjustment setpoints (T_{ch}*, RH*, v*) for the incubation environment parameters and the microscope parameters, and;
transmitting the selected adjustment setpoints (T_{ch}*, RH*) for the incubation environment parameters to at least one incubation control unit (212), said at least one incubation control unit (212) configured to supplying the incubation atmosphere based on the selected adjustment setpoints (T_{ch}*, RH*) for the incubation environment parameters.

14. An examination system (130) configured for microscopic examination of a sample (120) comprising a microscope (100), an incubation environment conditioning unit (110) connected to said microscope (100), and a user interface,
said microscope (100) comprising: an illumination optics (118), a microscope stage (116), an imaging optics (124), a sample chamber (106) configured to receive the sample, a microscope interface (108) configured for connection of the incubation environment conditioning unit (110) to the sample chamber (106), and a imaging optics chamber (122) separated from the sample chamber (106) and enclosing the imaging optics (118),
wherein the examination system (130) provides an incubation mode in which the sample chamber (106) is incubated by supply of an incubation atmosphere generated by said incubation environment conditioning unit (110), and
further comprising the controlling system (140) of any one of the preceding claims.

15. The examination system (130) of claim 14, wherein said user interface (142) comprises at least one of a display and input devices.

16. The examination system (130) claim 14 or 15, further configured to:
displaying to a user, via said user interface (142), current values of at least one of the group of: the at least one examination parameter (Ts) and of the at least one incubation environment parameter (RH).

17. The examination system (130) of any one of the claims 14 to 16, further comprising an atmosphere regulation module (216) for controlling the at least one incubation environment parameter (T_{ch}, RH) in the incubation mode.

18. The examination system (130) of claim 17, wherein said atmosphere regulation module (216) includes sensors for determining current values of the at least one incubation environment parameter (T_{ch}, RH).

19. The examination system (130) of any one of claims 14 to 18, said microscope (100) further comprising a microscope housing (102) enclosing the illumination optics (118), the microscope stage (116) and the imaging optics (124), and an integrated sample chamber (106) located within the microscope housing (102) and formed by a separated housing section (104) within said microscope housing (102),
wherein the imaging optics chamber (122) is located within said microscope housing (102) and separated from the sample chamber (106),
wherein said housing section (104) comprises said microscope interface (108) configured for connection of the incubation environment conditioning unit (110) to at least one of: the integrated sample chamber (106) and a stage top sample chamber (206) to be placed within said integrated sample chamber (106), and
wherein the examination system (130) provides a first incubation mode and a second incubation mode, wherein, in the first incubation mode, the integrated sample chamber (106) is incubated by supply of a first incubation atmosphere by said incubation environment conditioning unit (110), and, in the second incubation mode, the stage top chamber (206) is incubated by supply of a second incubation atmosphere by said incubation environment conditioning unit (110), and
wherein the controlling system (140) is configured to: operating the incubation environment conditioning unit (110) and the microscope (100) based on the selected adjustment setpoints for a selected one of the first incubation mode and the second incubation mode.

20. A method for microscopic examination of a sample (120) by means of an examination system (130) according to any one of claims 14 to 19, comprising the steps:
providing a sample (120) in the sample chamber (106);
providing the target setpoint (T_{S}**) of the at least one examination parameter, upon user input via said user interface (142); and
examining the sample (120) by means of the microscope (100) after defined threshold values (RH') of the predefined adjustment setpoints for the at least one incubation environment parameter of the incubation mode and for the at least one microscope parameter have been reached.

21. The method of claim 20, wherein the adjustment setpoints (T_{ch}*, RH*, v*) for the incubation environment parameters and the microscope parameters are selected from one or more datasets (300), each dataset comprising adjustment setpoints of one or more incubation environment parameters and/or microscope parameters correlated to target setpoints of the at least one examination parameter.

22. A method of operating an examination system (130) configured for microscopic examination of a sample (120), said examination system (130) comprising a microscope (100), an incubation environment conditioning unit (110) connected to said microscope (100), and a user interface (142),
said microscope (100) comprising: an illumination optics (118), a microscope stage (116), an imaging optics (124), a sample chamber (106) configured to receive the sample (120), a microscope interface (108) configured for connection of the incubation environment conditioning unit (110) to the sample chamber (106), and an imaging optics chamber (122) separated from the sample chamber (106) and enclosing the imaging optics (124),
wherein the examination system (130) provides an incubation mode in which the sample chamber (106) is incubated by supply of an incubation atmosphere generated by said incubation environment conditioning unit (110),
the method comprising the steps:
receiving (S1) a target setpoint (T_{S}**, CO₂**) of at least one examination parameter, preferably comprising at least a target temperature (T_{S}**) of the sample, upon user input via said user interface (142);
selecting (S2), based on the received target setpoint of the at least one examination parameter, predefined adjustment setpoints (T_{ch}*, RH*, v*) for at least one incubation environment parameter of the incubation mode and for at least one microscope parameter; and
operating (S3) the incubation environment conditioning unit (110) and the microscope (100) based on the selected adjustment setpoints.

23. The method of claim 22, operating the examination system (130) of any one of claims 14 to 19.

24. A computer program with a program code for performing the method of claim 22 or 23, when the computer program is run on a processor or on the controlling system (140) of any one of claims 1 to 13.
